# FASCICULE DE BREVET EUROPEEN

(11) **EP 1 347 738 B1**
(45) Date de publication et mention de la délivrance du brevet: **04.08.2010**
(21) Numéro de dépôt: 02711916.3
(22) Date de dépôt: 02.01.2002
(51) Int. Cl.: A61Q 5/12, A61K 8/41

(54) **COMPOSITION TRANSPARENTE DE TRAITEMENT COSMETIQUE DE TYPE EMULSION EAU-DANS-HUILE**
TRANSPARENTE KOSMETISCHE BEHANDLUNGSZUSAMMENSETZUNG AUS EINER ÖL-IN-WASSER-EMULSION
TRANSPARENT OIL-IN-WATER EMULSION COSMETIC TREATMENT COMPOSITION

(30) Priorité: 02.01.2001 FR 0100015; 02.07.2001 FR 0108759
(43) Date de publication de la demande: 01.10.2003
(73) Titulaire: L'Oréal, 75008 Paris (FR)
(72) Inventeur: FACK, Géraldine, F-92300 Levallois - Perret (FR); GAWTREY, Jonathan, F-92100 Boulogne-Billancout (FR); NICOLAS-MORGANTINI, Luc, F-60810 Rully (FR); RESTLE, Serge, F-95390 Saint-Prix (FR)
(74) Mandataire: Casalonga, Axel
(86) Numéro de dépôt international: PCT/FR2002/000004
(87) Numéro de publication internationale: WO 2002/053113

(56) Documents cités:
- EP-A- 0 331 833
- EP-A- 0 576 748
- EP-A- 0 595 683
- EP-A- 0 717 978
- EP-A- 0 782 846
- WO-A-01/28506
- WO-A-99/66883

## Description

La présente invention est relative à une composition transparente de traitement cosmétique des matières kératiniques, de type émulsion eau-dans-huile, et à un procédé de traitement cosmétique des matières kératiniques utilisant celle-ci.

Les émulsions de type eau-dans-huile sont bien connues dans le domaine des compositions cosmétiques.

Par exemple, le document WO 01/28506 décrit des compositions transparentes ou translucides sous la forme d'émulsions eau-dans-huile siliconée. Ces compositions comprennent:
(A) 0,01-10 % en poids d'au moins un tensioactif siliconé choisi parmi les copolymères siloxane/polyoxyalkylènes,
(B) 5-30 % en poids d'au moins une huile de silicone,
(C) 0,01-10 % en poids d'au moins un agent de conditionnement des cheveux azoté comme un tensioactif cationique de type ammonium quaternaire,
(D) au moins un tensioactif non ionique,
(E) de l'eau,
(F) au moins un polyol, et
(G) au moins un électrolyte.

Un autre exemple est décrit dans le document EP 595 683. Il divulgue des émulsions eau-dans-huile à phase continue fluorohydrocarbonée et comportant une phase aqueuse émulsionnée à l'aide d'un tensioactif siliconé. Des émulsions transparentes peuvent être obtenues en ajoutant des polyols en un pourcentage d'autant plus faible que le faux de fluor dans la phase grasse est élevée.

Toutefois, ces émulsions de type eau-dans-huile sont sensibles aux changements de températures et présentent alors des problèmes de stabilité selon la température environnante. En effet, on observe une nette séparation des phases aqueuse et huileuse à faible température et à température élevée.

Des émulions de type eau-dans-huile présentant une stabilité thermique sont déjà décrites dans le document EP 0 331 833 de Shiseido. Elles comprennent un tensioactif cationique, une phase huileuse et au moins un organopolysiloxane polyoxyéthyléné. Ces émulsions sont thermiquement stables grâce, entre autres, à l'addition d'un argile en une quantité de 0,2 à 5,0 % en poids par rapport au poids total de l'émulsion.

La demanderesse a découvert de manière surprenant qu'en utilisant dans certaines proportions, un composé choisi parmi les polyols et les électrolytes minéraux dans des émulsions de type eau-dans-huile, et notamment dans des après-shampoings, qui contiennent une huile siliconée volatile en une quantité inférieure à 15% en poids, un tensioactif siliconé et un tensioactif cationique, ce dernier étant contenu en une quantité supérieure à 0,5 % en poids par rapport à la composition, on améliorait la stabilité thermique desdites compositions.

En outre, l'utilisation d'un tel composé dans ces compositions permet d'obtenir, comme autre résultat intéressant, des émulsions eau-dans-huile transparentes. Cette utilisation permet donc d'améliorer aussi l'aspect des produits cosmétiques et de rendre le produit plus attractif pour le consommateur.

La présente invention a donc pour objet une composition transparente de traitement des matières kératiniques, de type émulsion eau-dans-huile, comprenant dans un milieu cosmétiquement acceptable, au moins un composé choisi parmi les polyols et les électrolytes minéraux, au moins une silicone volatile en une quantité inférieure à 15% en poids, au moins un tensioactif siliconé et au moins un tensioactif cationique en une concentration strictement supérieure à 0,5 % en poids par rapport au poids total de la composition.

Un autre objet de l'invention consiste en un procédé de traitement cosmétique des matières kératiniques mettant en oeuvre une composition transparente selon l'invention telle que décrite ci-dessous.

L'invention a encore pour objet une utilisation de la composition transparente selon l'invention comme après-shampoing.

D'autres objets, caractéristiques, aspects et avantages de l'invention apparaîtront encore plus clairement à la lecture de la description et des divers exemples qui suivent.

Selon l'invention, la composition transparente de traitement cosmétique des matières kératiniques, de type émulsion eau-dans-huile, comprend dans un milieu cosmétiquement acceptable, au moins un composé choisi parmi les polyols et les électrolytes minéraux, au moins une silicone volatile en une quantité inférieure à 15% en poids, au moins un tensioactif siliconé, et au moins un tensioactif cationique en une concentration strictement supérieure à 0,5 % en poids par rapport au poids total de la composition.

Par "composition transparente", on entend une composition présentant une turbidité inférieure ou égale à 300 NTU, les NTU étant les unités néphélométriques de mesure de la turbidité.

La turbidité peut être mesurée, par exemple, avec un turbidimètre Model 2100P commercialisé par la société HACH Company, les tubes utilisés pour la mesure étant référencés AR397A cat 24347-06.

L'étalonnage est effectué avec de la formazine et les mesures sont effectuées à température ambiante (20 à 25 °C). Les compositions de l'invention ont de préférence une turbidité allant de 0,05 à 100 NTU.

La composition transparente selon l'invention présente en particulier une force minimum de résistance à la pénétration de 0,075 N, telle que mesurée par pénétrométrie, et de préférence inférieure à 50 N, mieux encore inférieure à 5 N.

Les mesures de pénétrométrie ont été effectuées avec un analyseur de texture TA-TX2 (Rhéo). Elles correspondent à des mesures de force en compression dans les conditions suivantes :
- déplacement d'un disque (cylindre en ébonite de diamètre 13 mm) à la vitesse de 1 mm/s et détection de la force de résistance à la compression,
- pénétration dans le produit à la même vitesse que ci-dessus sur une profondeur de 10 mm,
- maintien de la compression dans le produit à cette profondeur pendant 300 s, et
- retrait de la sonde et détection de la force de rupture, à la vitesse de 1 mm/s.

Par polyol, on entend tout composé hydrocarboné en C₃-C₅₀, de préférence en C₃-C₂₀, comportant au moins deux groupes hydroxyles, et de préférence de 2 à 10 groupes hydroxyles.

Les polyols particulièrement préférés dans la présente invention sont choisis parmi les sucres tels que le sorbitol et la glycérine, et les alkylène-polyols en C₃-C₂₀, tels que le propylèneglycol, et les polyéthylèneglycols en C₈-C₂₀ tels que les PEG 300 et PEG 400.

La quantité de polyols est comprise de préférence dans l'intervalle allant de 15 à 55 % en poids, et notamment dans l'intervalle allant de 20 à 50 % par rapport au poids total de la composition.

Comme électrolytes minéraux, on peut utiliser ceux bien connus dans la technique. Les électrolytes utilisés de préférence dans la présente invention sont notamment les sels hydrosolubles de métaux monovalents ou divalents et d'un acide minéral.

Plus particulièrement, on préfère utiliser les électrolytes dont la solubilité dans l'eau est comprise entre 0,1 % en poids et 300 % en poids, et mieux encore entre 5 et 50 % en poids, par rapport au poids total de la solution aqueuse.

A titre d'exemples, on peut notamment citer le chlorure de sodium, le chlorure de potassium, le chlorure de calcium, le chlorure de magnésium, le sulfate de magnésium, les sels de sodium de l'acide phosphorique. De préférence, on utilise les sels de métaux monovalents et le chlorure de sodium est particulièrement préféré.

La quantité d'électrolytes minéraux est comprise de préférence dans l'intervalle allant de 0,1 à 20 % en poids, et notamment dans l'intervalle allant de 0,5 à 15 % par rapport au poids total de la composition.

Les proportions de polyol(s) et/ou d'électrolyte(s) sont telles que le rapport (polyol(s) et/ou électrolyte(s))/huiles est supérieur ou égal à 2, de préférence compris entre 2 et 10 et mieux encore compris entre 2 et 5.

Par huile, on entend dans la présente invention tout corps gras non miscible à l'eau, qui est liquide à température ambiante.

Selon la présente invention, les huiles comprennent au moins ladite silicone volatile, et peuvent comprendre en outre au moins l'un des composés choisis parmi les huiles végétales, les huiles animales, les huiles minérales, les huiles synthétiques, les esters d'acide gras, et leurs mélanges.

Comme huile végétale, on peut notamment mentionner l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum.

Comme huile animale, on peut notamment citer le perhydrosqualène.

Comme huile minérale, on peut notamment citer l'huile de paraffine et l'huile de vaseline.

A titre d'exemples d'huile synthétique, on peut citer le squalane, les poly(α-oléfines) comme l'isododécane ou l'isohexadécane, les huiles végétales transestérifiées, les huiles fluorées, et leurs mélanges.

Comme esters d'acide gras, on peut citer, par exemple, les composés de formule RₐCOOR_{b} dans laquelle Rₐ représente le reste d'un acide gras supérieur comportant de 6 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyl-dodécyle.

Les huiles sont contenues de préférence dans la composition selon l'invention en une quantité inférieure ou égale à 20 % en poids, mieux encore comprise entre 5 et 20 % en poids par rapport au poids total de la composition.

Les silicones volatiles utilisables dans l'invention sont des silicones linéaires ou cycliques, ayant une viscosité à température ambiante et sous pression atmosphérique, inférieure à 8 mm²/s (8 cSt).

La viscosité est mesurée de préférence par viscosimétrie capillaire, par exemple, à l'aide d'un viscosimètre capillaire, notamment de type Ubbelohde, à une température de 25 °C, selon la norme ASTM D445-97. On peut aussi utiliser la méthode dite de la chute de la bille.

Les silicones volatiles présentent généralement un point d'ébullition compris entre 60 °C et 260° C, et sont plus particulièrement choisies parmi :
(i) les silicones volatiles cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE^{®} 70045 V 2" par RHODIA, le décaméthylcyclopentasiloxane commercialisé sous le nom de "VOLATILE SILICONE 7158" par UNION CARBIDE, "SILBIONE^{®} 70045 V 5" par RHODIA ou sous le nom DC245 Fluid par DOW CORNING, ainsi que leurs mélanges.

On peut également citer les cyclocopolymères du type diméthylsiloxane/méthylalkylsiloxane, tel que la "SILICONE VOLATILE FZ 3109" commercialisée par la société UNION CARBIDE, de structure chimique :
avec D :

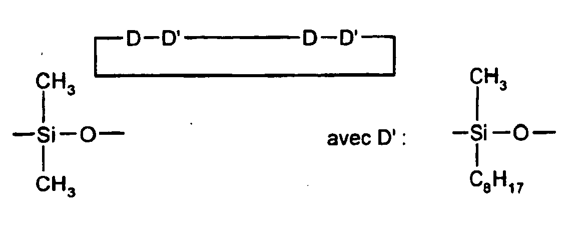

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopéntane ;
(ii) les silicones volatiles linéaires ayant 2 à 9 atomes de silicium et possédant une viscosité inférieure ou égale à 5 mm²/s à 25°C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

La composition selon l'invention comprend de préférence les silicones volatiles en une quantité comprise entre 5 et 15% en poids, et encore plus préférentiellement entre 8 et 15 % en poids par rapport au poids total de la composition.

Les tensioactifs siliconés utilisables dans la présente invention sont ceux bien connus de l'homme du métier. Ils peuvent être hydrosolubles, spontanément hydrodispersibles ou non hydrosolubles. De préférence, ils sont hydrosolubles ou spontanément hydrodispersibles.

Les tensioactifs siliconés sont, par exemple, choisis parmi les composés de formules générales (I), (II), (III), (IV) et (V) : 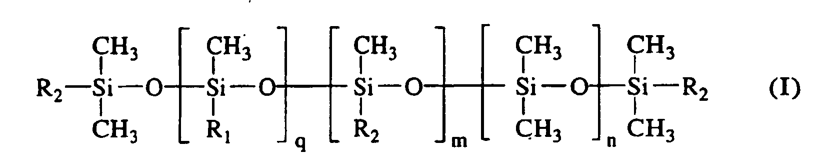 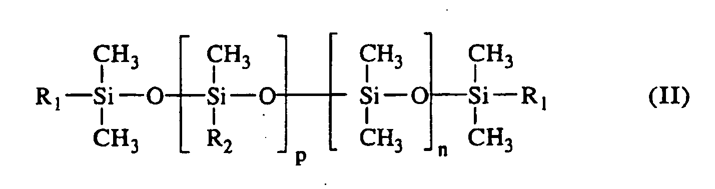 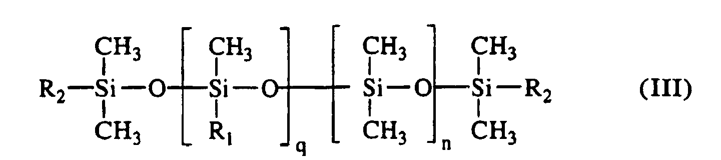 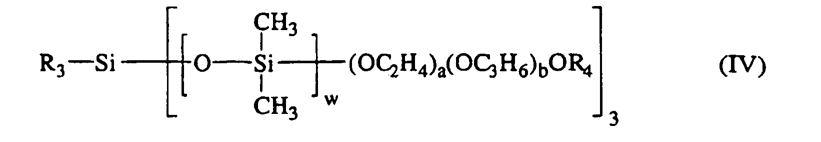 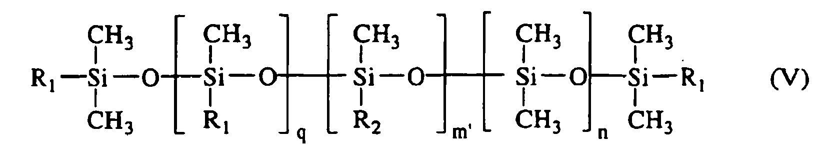 formules dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₃H₆O)_{b}-R₅ ou -C_{c}H_{2c}-O-(C₄H₈O)ₐ-R₅ ;
- R₃ et R₄, identiques ou différents, désignent chacun un groupe alkyle, linéaire ou ramifié, en C₁-C₁₂, et de préférence un groupe méthyle ;
- R₅, identique ou différent, est choisi parmi un atome d'hydrogène, un groupe alkyle, linéaire ou ramifié, comportant de 1 à 12 atomes de carbone, un groupe alcoxy, linéaire ou ramifié, comportant de 1 à 6 atomes de carbone, un groupe acyle, linéaire ou ramifié, comportant de 2 à 12 atomes de carbone, un groupe hydroxyle, -SO₃M, -OCOR₆, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle en C₁-C₁₂ éventuellement substitué sur l'amine et sur la chaîne alkyle, carboxyacyle en C₁-C₃₀, un groupement phosphono éventuellement substitué par un ou deux groupes aminoalkyle en C₁-C₁₂ substitués, -CO(CH₂)_{d}COOM, -OCOCHR₇(CH₂)_{d}COOM, -NHCO(CH₂)_{d}OH, -NH₃Y ;
- M, identique ou différent, désigne un atome d'hydrogène, Na, K, Li, NH₄ ou une amine organique ;
- R₆ désigne un groupe alkyle, linéaire ou ramifié, en C₁-C₃₀,
- R₇ désigne un atome d'hydrogène ou un groupe SO₃M ;
- d varie de 1 à 10 ;
- m varie de 0 à 20 ;
- m' varie de 1 à 20 ;
- n varie de 0 à 500 ;
- p varie de 1 à 50 ;
- q varie de 0 à 20 ;
- a varie de 0 à 50 ;
- b varie de 0 à 50 ;
- a + b est supérieur ou égal à 1 ;
- c varie de 0 à 4 ;
- w varie de 1 à 100 ;
- Y représente un anion minéral ou organique monovalent tel qu'un halogénure (chlorure, bromure), un sulfate, ou un carboxylate (acétate, lactate, citrate).

De préférence, on utilise des tensioactifs siliconés répondant aux formules générales (I) ou (II) telles que définies ci-dessus, et plus particulièrement, ceux répondant aux formules (I) ou (II) dans lesquelles au moins l'une des, et de préférence toutes les conditions suivantes sont satisfaites :
- c est égal à 2 ou 3 ;
- R₁ désigne le groupe méthyle ;
- R₅ représente un atome d'hydrogène, un groupe méthyle ou un groupe acétyle et de préférence un atome d'hydrogène ;
- a varie de 1 à 25 et plus particulièrement de 2 à 25 ;
- b varie de 0 à 25, de préférence de 10 à 20 ;
- n varie de 0 à 100;
- p varie de 1 à 20.

Les tensioactifs siliconés les plus particulièrement préférés sont, par exemple, ceux vendus sous les dénominations commerciales FLUID DC 193 et DC 5225C par la société DOW CORNING, SILWET^{®} L 77 par la société OSI et MAZIL^{®} 756 par la société MAZER PPG.

Les tensioactifs siliconés sont contenus dans la présente invention en une quantité comprise entre 0,01 et 10 % en poids, mieux encore comprise entre 0,1 et 5 % en poids, et encore plus préférentiellement entre 0,2 et 3 % en poids par rapport au poids total de la composition de traitement des matières kératiniques.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :
- ceux qui présentent la formule générale (VI) suivante : 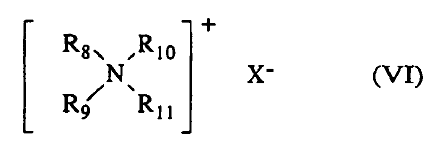 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (VII) suivante : 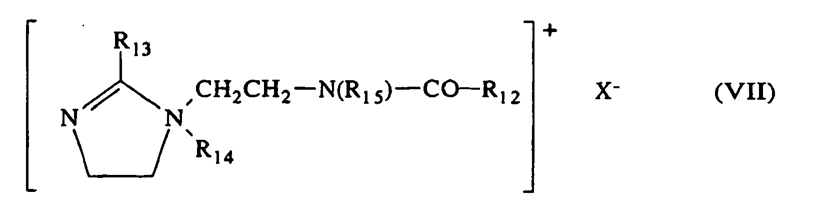 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X est un anion choisi dans le groupe des halogénures, phosphates, acétates, lactates, alkylsulfates, alkyl- ou alkylaryl-sulfonates De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (VIII) : 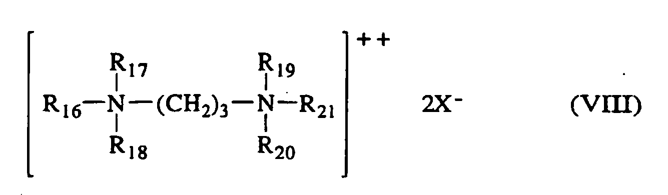 dans laquelle R₁₆ désigne un radical aliphatique comportant environ de 16 à 30 atomes de carbone, R₁₇, R₁₈, R₁₉, R₂₀ et R₂₁ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IX) suivante : 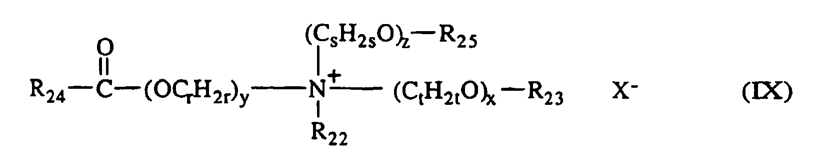 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
      - le radical 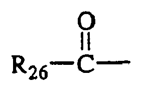
      - les radicaux R₂₇ hydrocarbonés en C₁-C₂₂, linéaires ou ramifiés, saturés ou insaturés,
      - l'atome d'hydrogène,
   R₂₅ est choisi parmi :
      - le radical 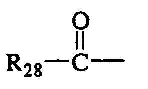
      - les radicaux R₂₉ hydrocarbonés en C₁-C₆, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1. Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IX) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
- le radical 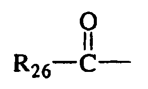
- les radicaux méthyle, éthyle ou hydrocarbonés en C₁₄-C₂₂,
- l'atome d'hydrogène ;
- R₂₅ est choisi parmi :
- le radical 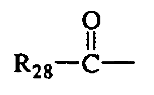
- l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IX) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire, on préfère en particulier ceux qui répondent à la formule (VI) dans laquelle :
R₈ représente un groupe alkyle en C₁₂₋₃₀, de préférence en C₁₄₋₂₂, alcényle en C₁₂₋₃₀, alkyl(C₁₂-C₂₂)amidoalkyle(C₂-C₆), alkyl(C₁₂-C₂₂)-acétate, ou un groupe aromatique tel qu'aryle ou alkylaryle en C₆-C₁₂,
R₉ à R₁₁, qui peuvent être identiques ou différents, représentent un groupe alkyle en C₁₋₈, alcényle en C₁₋₈, alcoxy en C₁₋₈, hydroxyalkyle en C₁₋₈, polyoxyalkylène (C₂-C₆) ou alkylamide en C₁₋₈ ; et
X est un anion choisi dans le groupe des halogénures, phosphates, acétates, lactates, alkyl(C₂-C₆)sulfates, alkyl- ou alkylaryl-sulfonates.

A titre d'exemples de ces composés particulièrement préférés, on peut notamment citer d'une part, les sels de tétraalkylammonium, notamment les chlorures de tétraalkylammonium comme, par exemple, les chlorures de dialkyldiméthylammonium ou d'alkyltriméthylammonium dans lesquels le radical alkyle comporte environ de 12 à 30 atomes de carbone, en particulier les chlorures de distéaryldiméthylammonium, de béhényltriméthylammonium, d'arachidyltriméthylammonium, de stéaryltriméthylammonium, de cétyltriméthylammonium, de benzyldiméthylstéarylammonium ou encore, d'autre part, les sels d'alkyl(C₈-C₃₀)amidoalkyl(C₂-C₆)triméthylammonium, notamment le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium et le chlorure de palmitylamidopropyltriméthylammonium.

La composition de traitement cosmétique des matières kératiniques comprend de préférence le ou les tensioactifs cationiques en une quantité comprise entre 0,5 et 10 % en poids, mieux encore entre 0,8 et 8 % en poids, et encore plus préférentiellement entre 1 et 5 % en poids par rapport au poids total de la composition.

Par "milieu cosmétiquement acceptable", on entend un milieu compatible avec toutes les matières kétatiniques telles que la peau, les cheveux, les ongles, les cils, les sourcils, les lèvres et toute autre zone du corps et du visage, mais aussi d'odeur, d'aspect et de toucher agréables.

Le milieu cosmétiquement acceptable comprend l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les alcanes en C₅-C₁₀ ; les cétones en C₃₋₄ comme l'acétone et la méthyléthylcétone ; les acétates d'alkyle en C₁-C₄ comme l'acétate de méthyle, l'acétate d'éthyle et l'acétate de butyle ; le diméthoxyéthane, le diéthoxyéthane ; et leurs mélanges.

Le pH des compositions de l'invention est compris entre 3 et 12, de préférence entre 4 et 8.

Les compositions transparentes selon l'invention peuvent également contenir des additifs tels que des polymères cationiques, anioniques, non ioniques ou amphotères, des silicones non volatiles, modifiées ou non, des épaississants polymériques naturels ou synthétiques, anioniques, amphotères, zwittérioniques, non ioniques ou cationiques, associatifs ou non, des filtres solaires, des parfums, des colorants, des particules organiques ou minérales, des conservateurs, des agents de stabilisation du pH.

L'homme du métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 50 % en poids par rapport au poids total de la composition.

Les compositions selon l'invention peuvent se présenter sous forme de liquides fluides ou épaissis, de gels, de crèmes, ou d'émulsions simples ou multiples.

Les compositions peuvent être utilisées, par exemple, comme shampoings, après-shampoings, produits de coloration ou de décoloration ou de permanente, produits de coiffage, soins rincés, masques de soin profond, gels douches, lotions ou crèmes de traitement du cuir chevelu, produits de rasage ou produits d'épilation.

La présente invention concerne également un procédé de traitement cosmétique des matières kératiniques qui consiste à appliquer une quantité efficace d'une composition transparente telle que décrite ci-dessus, sur les matières kératiniques, à effectuer un éventuel rinçage après un éventuel temps de pose.

Selon un mode de réalisation préféré de l'invention, la composition transparente peut être utilisée comme après-shampoing.

Les exemples suivants illustrent la présente invention et ne doivent être considérés en aucune manière comme limitant l'invention.

### EXEMPLES

On a préparé des compositions transparentes d'après-shampoing à partir des ingrédients indiqués dans le tableau ci-dessous. Les teneurs indiquées sont exprimées en % en poids par rapport au poids total de la composition.

| | Ex. 1 | Ex. 2 | Ex. 3 |
|---|---|---|---|
| Cyclopentadiméthylsiloxane⁽¹⁾ | 7 | 5,4 | 11,5 |
| Myristate d'isopropyle | - | 2,1 | - |
| Polydiméthyl/méthylsiloxane (18 moles d'oxyde d'éthylène/ 18 moles d'oxyde de propylène)⁽²⁾ - DC 5225C de DOW CORNING | 0,5 MA | 0,5 MA | 0,5 MA |
| Chlorure de palmitylamidopropyltriméthyl-ammonium à 60 % de MA dans le propylèneglycol - Varisoft PATC de Goldschmidt | 1,2 MA | 2,7 MA | 1,2 MA |
| Propylèneglycol | 0,8 | 7,3 | 3,2 |
| NaCl | - | - | 9,7 |
| Saccharose | - | 29,5 | 19,35 |
| Glycérine | 40,8 | - | 1,2 |
| Eau | 100 | 100 | 100 |

| | | | |
|---|---|---|---|
| MA : Matières actives ⁽¹⁾ DC 245 de DOW CORNING ⁽²⁾ à 10 % dans du cyclopentadiméthylsiloxane. | | | |

On a appliqué les compositions sur les cheveux et rincé après un temps de pose d'une minute, le rinçage étant aisé. On a ensuite fait sécher les cheveux.

Les cheveux séchés sont doux et déliés au toucher, et sans résidus désagréables.

Ces compositions sont stables 2 mois à température ambiante et à 45 °C.

Les turbidités des compositions des exemples 1 et 2 sont respectivement de 75 NTU et de 90 NTU.

## Revendications

1. Composition transparente de traitement cosmétique des matières kératiniques, **caractérisée en ce qu'**elle se présente sous la forme d'une émulsion eau-dans-huile, **en ce qu'**elle comprend dans un milieu cosmétiquement acceptable, au moins un composé choisi parmi les polyols et les électrolytes minéraux, au moins une silicone volatile n une quantité inférieure à 15% en poids par rapport au poids total de la composition, au moins un tensioactif siliconé, et au moins un tensioactif cationique en une concentration supérieure à 0,5 % en poids par rapport au poids total de la composition, et **en ce que** le rapport (polyol(s) et/ou électrolyte(s))/huiles est supérieur ou égal à 2, les huiles comprenant au moins ladite silicone volatile.

2. Composition transparente de traitement cosmétique des matières kératiniques selon la revendication 1, **caractérisée en ce que** le rapport (polyol(s) et/ou électrolyte(s))/huiles est compris entre 2 et 10.

3. Composition transparente de traitement cosmétique des matières kératiniques selon la revendication 2, **caractérisée en ce que** le rapport (polyol(s) et/ou électrolyte(s))/huiles est compris entre 2 et 5.

4. Composition transparente de traitement cosmétique des matières kératiniques selon l'une quelconque des revendications précédentes, **caractérisée en ce que** les huiles comprennent en outre au moins l'un des composés choisis parmi les huiles végétales, les huiles animales, les huiles minérales, les huiles synthétiques, les esters d'acide gras, et leurs mélanges.

5. Composition transparente de traitement cosmétique des matières kératiniques selon l'une quelconque des revendications précédentes, **caractérisée en ce que** les huiles sont contenues en une quantité inférieure ou égale à 20 % en poids par rapport au poids total de la composition.

6. Composition transparente de traitement cosmétique des matières kératiniques selon la revendication 5, **caractérisée en ce que** les huiles sont contenues en une quantité comprise entre 5 et 20 % en poids par rapport au poids total de la composition.

7. Composition transparente de traitement cosmétique des matières kératiniques selon l'une quelconque des revendications précédentes, **caractérisée en ce que** les polyols sont choisis parmi les sucres et les alkylène-polyols en C₃-C₂₀.

8. Composition transparente de traitement cosmétique des matières kératiniques selon la revendication 7, **caractérisée en ce que** les polyols sont choisis parmi la glycérine, le sorbitol, le propylèneglycol et les polyéthylèneglycols en C₈-C₂₀.

9. Composition transparente de traitement cosmétique des matières kératiniques selon l'une quelconque des revendications précédentes, **caractérisée en ce que** les polyols sont contenus en une quantité allant de 15 à 55 % en poids par rapport au poids total de la composition.

10. Composition transparente de traitement cosmétique des matières kératiniques selon la revendication 9, **caractérisée en ce que** les polyols sont contenus en une quantité allant de 20 à 50 % en poids par rapport au poids total de la composition.

11. Composition transparente de traitement cosmétique des matières kératiniques selon l'une quelconque des revendications précédentes, **caractérisée en ce que** les électrolytes minéraux sont choisis parmi le chlorure de sodium, le chlorure de potassium, le chlorure de calcium, le chlorure de magnésium, le sulfate de magnésium, et les sels de sodium de l'acide phosphorique.

12. Composition transparente de traitement cosmétique des matières kératiniques selon l'une quelconque des revendications précédentes, **caractérisé en ce que** les électrolytes minéraux sont contenus en une quantité allant de 0,1 à 20 % en poids par rapport au poids total de la composition.

13. Composition transparente de traitement cosmétique des matières kératiniques selon la revendication 12, **caractérisé en ce que** les électrolytes minéraux sont contenus en une quantité allant de 0,5 à 15 % en poids par rapport au poids total de la composition.

14. Composition transparente de traitement cosmétique des matières kératiniques selon l'une quelconque des revendications précédentes, **caractérisée en ce que** les silicones volatiles sont des silicones linéaires ou cycliques, ayant une viscosité à température ambiante et sous pression atmosphérique, inférieure à 8 mm²/s.

15. Composition transparente de traitement cosmétique des matières kératiniques selon la revendication 14, **caractérisée en ce que** les silicones volatiles sont choisies parmi :
- les silicones volatiles cycliques comportant de 3 à 7 atomes de silicium, et
- les silicones volatiles linéaires ayant 2 à 9 atomes de silicium et possédant une viscosité inférieure ou égale à 5 mm²/s à 25 °C.

16. Composition transparente de traitement cosmétique des matières kératiniques selon l'une quelconque des revendications précédentes, **caractérisée en ce qu'**elle comprend les silicones volatiles en une quantité comprise entre 5 et 15 % en poids, par rapport au poids total de la composition.

17. Composition transparente de traitement cosmétique des matières kératiniques selon la revendication 16. **caractérisée en ce qu'**elle comprend les silicones volatiles en une quantité comprise entre 8 et 15 % en poids, par rapport au poids total de la composition.

18. Composition transparente de traitement cosmétique des matières kératiniques selon l'une quelconque des revendications précédentes, **caractérisée** on ce que les tensioactifs silicones sont choisis parmi les composés de formules générales (I). (II). (III), (IV) et (V): 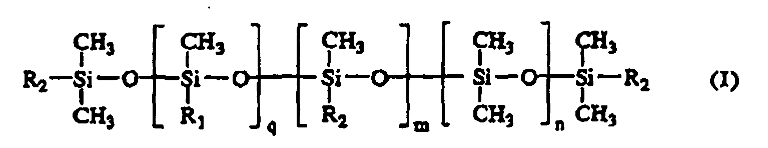 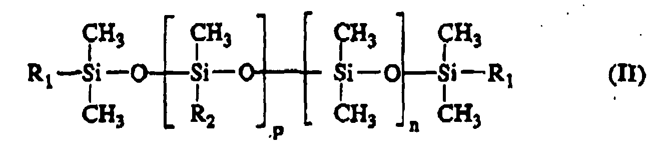 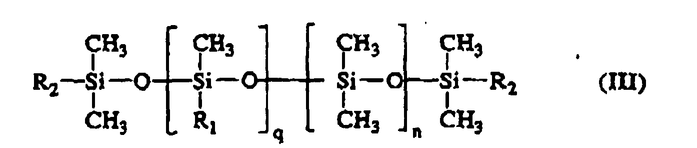 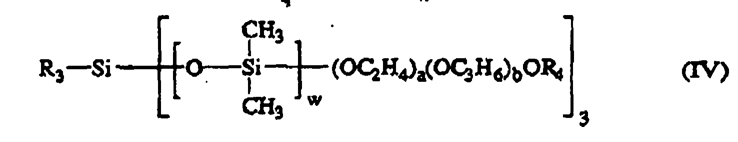 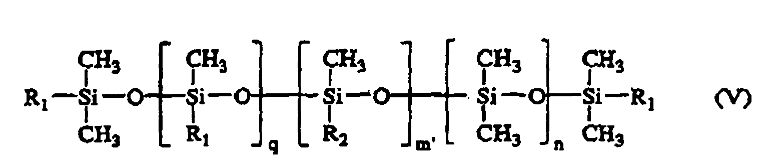 formules dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₅H₆O)ₐ-R₅ ou -C_{c}H_{2c}-O-(C₄H₈O)ₐ-R₅ ;
- R₃ et R₄, identiques ou différents, désignent chacun un groupe alkyle, linéaire ou ramifié, en C₁-C₁₂, et de préférence un groupe méthyle ;
- R₅, identique ou différent, est choisi parmi un atome d'hydrogène, un groupe alkyle, linéaire ou ramifié, comportant de 1 à 12 atomes de carbone, un groupe alcoxy, linéaire ou ramifié, comportant de 1 à 6 atomes de carbone, un groupe acyle, linéaire ou ramifié, comportant de 2 à 12 atomes de carbone, un groupe hydroxyle, -SO₃M, -OCOR₆, aminoalcoxy en C₁-C₆ éventuellement substitué sur l'amine, aminoacyle en C₂-C₆ éventuellement substitué sur l'aminé, -NHCH₂CH₂COOM, -N(CH₂CH₂COOM)₂, aminoalkyle en C₁-C₁₂ éventuellement substitué sur l'amine et sur la chaîne alkyle, carboxyacyle en C₁-C₃₀, un groupement phosphono éventuellement substitué par un ou deux groupes aminoalkyle en C₁-C₁₂ substitués, -CO(CH₂)ₐCOOM, -OCOCHR,(CH₂)₄COOM, -NHCO(CH₂)₄OH, -NH,Y ;
- M, identique ou différent, désigne un atome d'hydrogène, Na, K, Li, NH₄ ou une amine organique ;
- R₆ désigne un groupe alkyle, linéaire ou ramifié, en C₁-C₃₀ ;
- R₇ désigne un atome d'hydrogène ou un groupe SO₃M ;
- d varie de 1 à 10 ;
- m varie de 0 à 20 ;
- m' varie de 1 à 20 ;
- n varie de 0 à 500 ;
- p varie de 1 à 50 ;
- q varie de 0 à 20 ;
- a varie de 0 à 50 ;
- b varie de 0 à 50 ;
- a + b est supérieur ou égal à 1 ;
- c varie de 0 à 4 ;
- w varie de 1 à 100 ;
- Y représente un anion minéral ou organique monovalent tel qu'un halogénure, un sulfate, ou un carboxylate.

19. Composition transparente de traitement cosmétique des matières kératiniques selon la revendication 18, **caractérisée en ce** les tensioactifs siliconés sont choisis parmi ceux répondant aux formules (I) ou (II) dans lesquelles au moins l'une des, et de préférence toutes les conditions suivantes sont satisfaites :
- c est égal à 2 ou 3 ;
- Rₜ désigne le groupe méthyle ;
- R₅ représente un atome d'hydrogène, un groupe méthyle ou un groupe acétyle et de préférence un atome d'hydrogène ;
- a varie de 1 à 25 et plus particulièrement de 2 à 25 ;
- b varie de 0 a 25 :
- n varie de 0 à 100;
- p varie de 1 à 20.

20. Composition transparente de traitement cosmétique des matières kératiniques selon l'une quelconque des revendications précédentes, **caractérisée en ce qu'**elle comprend les tensioactifs siliconés en une quantité comprise entre 0,01 et 10 % en poids par rapport au poids total de la composition.

21. Composition transparente de traitement cosmétique des matières kératiniques selon la revendication 20, **caractérisée en ce qu'**elle comprend les tensioactifs siliconés en une quantité comprise entre 0,1 et 5 % en poids, par rapport au poids total de la composition.

22. Composition transparente de traitement cosmétique des matières kératiniques selon la revendication 21, **caractérisée en ce qu'**elle comprend les tensioactifs siliconés en une quantité comprise entre 0,2 et 3 % en poids, par rapport au poids total de la composition.

23. Composition transparente de traitement cosmétique des matières kératiniques selon l'une quelconque des revendications précédentes, **caractérisée en ce que** les tensioactifs cationiques sont choisis parmi les sels d'amines grasses primaires, secondaires ou tertiaires, éventuellement polyoxyalkylénées, les sels, d'ammonium quaternaire et leurs mélanges.

24. Composition transparente de traitement cosmétique des matières kératiniques selon la revendication 23, **caractérisée en ce que** les sels d'ammonium quaternaire sont choisis parmi :
- ceux qui présentent la formule générale (VI) suivante : 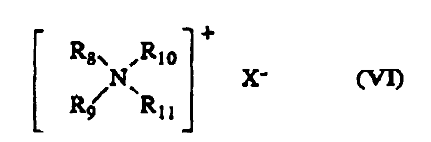 dans laquelle les radicaux R₅ à R₁₁, qui peuvent être identiques ou différents, représentent un radical aliphatique, linéaire ou ramifié, comportant de 1 à 30 atomes de carbone, ou un radical aromatique tel que aryle ou alkylaryle ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (VIII) : 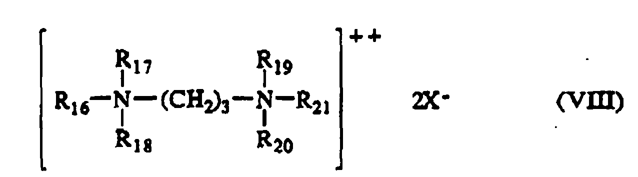 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates ;
- les sels d'ammonium quaternaire contenant au moins une fonction ester.

25. Composition transparente de traitement cosmétique des matières kératiniques selon la revendication 23 ou 24, **caractérisée en ce** les sels d'ammonium quaternaire sont choisis parmi :
- ceux qui présentent la formule générales (VI) suivante : 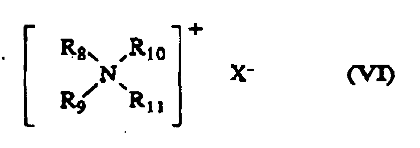 dans laquelle ;
R₈ représente un groupe alkyle en C₁₂₋₃₀, de préférence en C₁₄₋₂₂, alcényle en C₁₂₋₃₀, alkyl(C₁₂-C₂₂)amidoalkyle(C₂-C₆), alkyl(C₁₂-C₂₂)-acétate, ou un groupe aromatique tel qu'aryle ou alkylaryle en C₆-C₁₂, R₉ à R₁₁, qui peuvent être identiques ou différents, représentent un groupe alkyle en C₁₋₈, alcényle en C₁₋₈, alcoxy en C₁₋₈, hydroxyalkyle en C₁₋₈, polyoxyalkylène (C₂-C₆) ou alkylamide en C₁₋₈;
X est un anion choisi dans le groupe des halogénures, phosphates, acétates, lactates, alkyl(C₂-C₆)sulfates, alkyl- ou alkylaryl-sulfonates.

26. Composition transparente de traitement cosmétique des matières kératiniques selon l'une quelconque des revendications précédentes, **caractérisée en ce qu'**elle comprend les tensioactifs cationiques en une quantité comprise entre 0,5 et 10 % en poids par rapport au poids total de la composition.

27. Composition transparente de traitement cosmétique des matières kératiniques selon la revendication 26, **caractérisée en ce qu'**elle comprend les tensioactifs cationiques en une quantité comprise entre 0,8 et 8 % en poids par rapport au poids total de la composition.

28. Composition transparente de traitement cosmétique des matières kératiniques selon la revendication 27, **caractérisée en ce qu'**elle comprend les tensioactifs cationiques en une quantité comprise entre 1 et 5 % en poids, par rapport au poids total de la composition.

29. Composition transparente de traitement cosmétique des matières kératiniques selon l'une quelconque des revendications précédentes, **caractérisée en ce que** le milieu cosmétiquement acceptable comprend l'eau ou un mélange d'eau et d'un solvant cosmétiquement acceptable.

30. Composition transparente de traitement cosmétique des matières kératiniques selon la revendication 29, **caractérisée en ce que** le solvant cosmétiquement acceptable est choisi parmi les alcools inférieurs en C₁-C₄, les alcanes en C₅-C₁₀, les cétones en C₃₋₄, les acétates d'alkyle en C₁-C₄, le diméthoxyéthane, le diéthoxyéthane, et leurs mélanges.

31. Composition transparente de traitement cosmétique des matières kératiniques selon l'une quelconque des revendications précédentes, **caractérisée en ce qu'**elle comprend des additifs tels que des polymères cationiques, anioniques, non ioniques ou amphotères, des silicones non volatiles, modifiées ou non, des épaississants polymériques naturels ou synthétiques, anioniques, amphotères, zwittérioniques, non ioniques ou cationiques, associatifs ou non, des filtres solaires, des parfums, des colorants, des particules organiques ou minérales, des conservateurs, des agents de stabilisation du pH.

32. Procédé de traitement cosmétique des matières kératiniques, **caractérisé en ce que** l'on applique sur les matières kératiniques une composition transparente de traitement cosmétique selon l'une quelconque des revendications précédentes.

33. Utilisation d'une composition transparente de traitement cosmétique selon l'une quelconque des revendications 1 à 31, comme après-shampoing.

## Claims

1. Transparent composition for the cosmetic treatment of keratin materials, **characterized in that** it is in the form of a water-in-oil emulsion, **in that** it comprises, in a cosmetically acceptable medium, at least one compound chosen from polyols and mineral electrolytes, at least one volatile silicone in an amount of less than 15% by weight relative to the total weight of the composition, at least one silicone surfactant, and at least one cationic surfactant in a concentration greater than 0.5% by weight relative to the total weight of the composition, and **in that** the (polyol(s) and/or electrolyte(s))/oils ratio is greater than or equal to 2, the oils comprising at least said volatile silicone.

2. Transparent composition for the cosmetic treatment of keratin materials according to Claim 1, **characterized in that** the (polyol(s) and/or electrolyte(s))/oils ratio is between 2 and 10.

3. Transparent composition for the cosmetic treatment of keratin materials according to Claim 2, **characterized in that** the (polyol(s) and/or electrolyte(s))/oils ratio is between 2 and 5.

4. Transparent composition for the cosmetic treatment of keratin materials according to any one of the preceding claims, **characterized in that** the oils comprise, in addition, at least one of the compounds chosen from plant oils, animal oils, mineral oils, synthetic oils, fatty acid esters, and mixtures thereof.

5. Transparent composition for the cosmetic treatment of keratin materials according to any one of the preceding claims, **characterized in that** the oils are incorporated in an amount less than or equal to 20% by weight relative to the total weight of the composition.

6. Transparent composition for the cosmetic treatment of keratin materials according to Claim 5, **characterized in that** the oils are incorporated in an amount between 5 and 20% by weight relative to the total weight of the composition.

7. Transparent composition for the cosmetic treatment of keratin materials according to any one of the preceding claims, **characterized in that** the polyols are chosen from sugars and C₃-C₂₀ alkylene polyols.

8. Transparent composition for the cosmetic treatment of keratin materials according to Claim 7, **characterized in that** the polyols are chosen from glycerol, sorbitol, propylene glycol and C₈-C₂₀ polyethylene glycols.

9. Transparent composition for the cosmetic treatment of keratin materials according to any one of the preceding claims, **characterized in that** the polyols are incorporated in an amount ranging from 15 to 55% by weight relative to the total weight of the composition.

10. Transparent composition for the cosmetic treatment of keratin materials according to Claim 9, **characterized in that** the polyols are incorporated in an amount ranging from 20 to 50% by weight relative to the total weight of the composition.

11. Transparent composition for the cosmetic treatment of keratin materials according to any one of the preceding claims, **characterized in that** the mineral electrolytes are chosen from sodium chloride, potassium chloride, calcium chloride, magnesium chloride, magnesium sulphate, and the sodium salts of phosphoric acid.

12. Transparent composition for the cosmetic treatment of keratin materials according to any one of the preceding claims, **characterized in that** the mineral electrolytes are incorporated in an amount ranging from 0.1 to 20% by weight relative to the total weight of the composition.

13. Transparent composition for the cosmetic treatment of keratin materials according to Claim 12, **characterized in that** the mineral electrolytes are incorporated in an amount ranging from 0.5 to 15% by weight relative to the total weight of the composition.

14. Transparent composition for the cosmetic treatment of keratin materials according to any one of the preceding claims, **characterized in that** the volatile silicones are linear or cyclic silicones, having a viscosity at ambient temperature and at atmospheric pressure of less than 8 mm²/s.

15. Transparent composition for the cosmetic treatment of keratin materials according to Claim 14, **characterized in that** the volatile silicones are chosen from:
- cyclic volatile silicones comprising from 3 to 7 silicon atoms; and
- linear volatile silicones having 2 to 9 silicon atoms and possessing a viscosity of less than or equal to 5 mm²/s at 25°C.

16. Transparent composition for the cosmetic treatment of keratin materials according to any one of the preceding claims, **characterized in that** it comprises volatile silicones in an amount between 5 and 15% by weight, relative to the total weight of the composition.

17. Transparent composition for the cosmetic treatment of keratin materials according to Claim 16, **characterized in that** it comprises the volatile silicones in an amount between 8 and 15% by weight, relative to the total weight of the composition.

18. Transparent composition for the cosmetic treatment of keratin materials according to any one of the preceding claims, **characterized in that** the silicone surfactants are chosen from the compounds of general formulae (I), (II), (III), (IV) and (V): 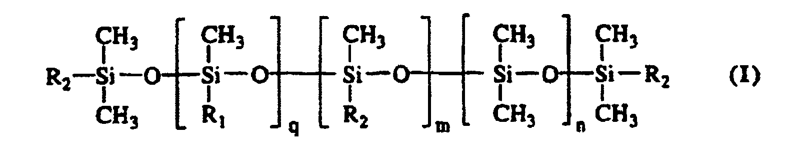 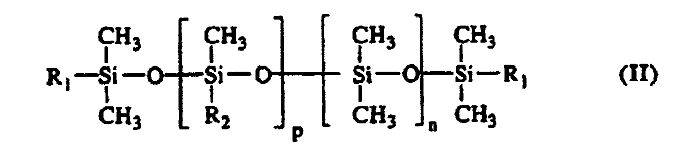 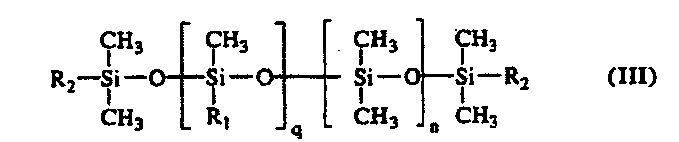 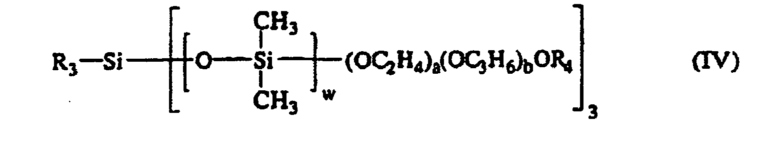 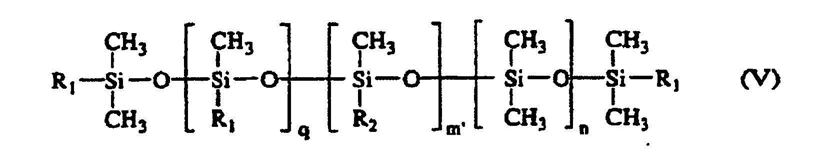 in which formulae:
- R₁, which may be identical or different, represents a linear or branched C₁-C₃₀ alkyl group, or phenyl group;
- R₂, which may be identical or different, represents -C_{c}H_{2c}-O-(C₂H₄O)ₐ-(C₃H₆O)_{b}-R₅ or -C_{c}H_{2c}-O-(C₄H₈O)ₐ-R₅;
- R₃ and R₄, which may be identical or different, each denote a linear or branched C₁-C₁₂ alkyl group, and preferably a methyl group;
- R₅, which may be identical or different, is chosen from a hydrogen atom, a linear or branched alkyl group containing from 1 to 12 carbon atoms, a linear or branched alkoxy group containing from 1 to 6 carbon atoms, a linear or branched acyl group containing from 2 to 12 carbon atoms, a hydroxyl group, -SO₃M, -OCOR₆, C₁-C₆ aminoalkoxy optionally substituted on the amine, C₂-C₆ aminoacyl optionally substituted on the amine, -NHCH₂CH₂COOM, -N(CH₂CH₂COOM)₂, C₁-C₁₂ aminoalkyl optionally substituted on the amine and on the alkyl chain, C₁-C₃₀ carboxyacyl, a phosphono group optionally substituted with one or two substituted C₁-C₁₂ aminoalkyl groups, -CO(CH₂)_{d}COOM, -OCOCHR₇(CH₂)_{d}COOM, -NHCO(CH₂)_{d}OH, -NH₃Y;
- M, which may be identical or different, denotes a hydrogen atom, Na, K, Li, NH₄ or an organic amine;
- R₆ denotes a linear or branched C₁-C₃₀ alkyl group;
- R₇ denotes a hydrogen atom or an SO₃M group;
- d ranges from 1 to 10;
- m ranges from 0 to 20;
- m' ranges from 1 to 20;
- n ranges from 0 to 500;
- p ranges from 1 to 50;
- q ranges from 0 to 20;
- a ranges from 0 to 50;
- b ranges from 0 to 50;
- a + b is greater than or equal to 1;
- c ranges from 0 to 4;
- w ranges from 1 to 100;
- Y represents a monovalent, mineral or organic anion such as a halide, a sulphate or a carboxylate.

19. Transparent composition for the cosmetic treatment of keratin materials according to Claim 18, **characterized in that** the silicone surfactants are chosen from those corresponding to formula (I) or (II) in which at least one and preferably all of the following conditions are satisfied:
- c is equal to 2 or 3;
- R₁ denotes the methyl group;
- R₅ represents a hydrogen atom, a methyl group or an acetyl group and preferably a hydrogen atom;
- a ranges from 1 to 25 and more particularly from 2 to 25;
- b ranges from 0 to 25;
- n ranges from 0 to 100;
- p ranges from 1 to 20.

20. Transparent composition for the cosmetic treatment of keratin materials according to any one of the preceding claims, **characterized in that** it comprises the silicone surfactants in an amount of between 0.01 and 10% by weight relative to the total weight of the composition.

21. Transparent composition for the cosmetic treatment of keratin materials according to Claim 20, **characterized in that** it comprises the silicone surfactants in an amount of between 0.1 and 5% by weight relative to the total weight of the composition.

22. Transparent composition for the cosmetic treatment of keratin materials according to Claim 21, **characterized in that** it comprises the silicone surfactants in an amount of between 0.2 and 3% by weight relative to the total weight of the composition.

23. Transparent composition for the cosmetic treatment of keratin materials according to any one of the preceding claims, **characterized in that** the cationic surfactants are chosen from optionally polyoxyalkylenated primary, secondary and tertiary fatty amine salts, quaternary ammonium salts and mixtures thereof.

24. Transparent composition for the cosmetic treatment of keratin materials according to Claim 23, **characterized in that** the quaternary ammonium salts are chosen from:
- those having the general formula (VI) below: 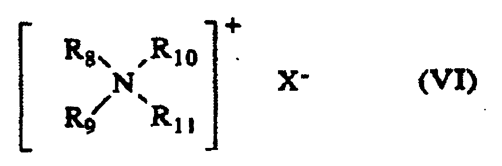 in which the radicals R₈ to R₁₁, which may be identical or different, represent a linear or branched aliphatic radical containing from 1 to 30 carbon atoms, or an aromatic radical such as an aryl or alkylaryl radical; X is an anion chosen from the group of halides, phosphates, acetates, lactates, (C₂-C₆)alkyl sulphates and alkyl- or alkylaryl- sulphonates;
- the quaternary ammonium salts of imidazoline;
- the diquaternary ammonium salts of formula (VIII): 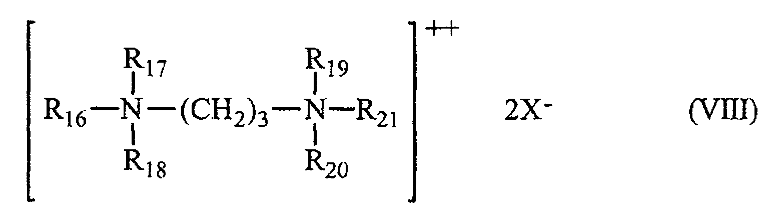 in which R₁₆ denotes an aliphatic radical containing approximately from 16 to 30 carbon atoms, R₁₇, R₁₈, R₁₉, R₂₀ and R₂₁, which may be identical or different, are chosen from hydrogen or an alkyl radical containing from 1 to 4 carbon atoms, and X is an anion chosen from the group of halides, acetates, phosphates, nitrates and methyl sulphates;
- quaternary ammonium salts containing at least one ester function.

25. Transparent composition for the cosmetic treatment of keratin materials according to Claim 23 or 24, **characterized in that** the quaternary ammonium salts are chosen from:
- those having the general formula (VI) below: 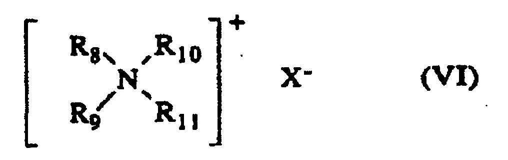 in which:
R₈ represents a C₁₂₋₃₀ and preferably C₁₄₋₂₂ alkyl group, a C₁₂₋₃₀ alkenyl group, a (C₁₂-C₂₂) alkylamido (C₂-C₆) alkyl group, a (C₁₂-C₂₂)alkylacetate group, or an aromatic group such as C₆-C₁₂ aryl or alkylaryl group,
R₉ to R₁₁, which may be identical or different, represent a C₁₋₈ alkyl, C₁₋₈ alkenyl, C₁₋₈ alkoxy, C₁₋₈ hydroxyalkyl, polyoxy(C₂-C₆)alkylene or C₁₋₈ alkylamide group,
X is an anion chosen from halides, phosphates, acetates, lactates, (C₂-C₆)alkyl sulphates and alkyl- or alkylaryl- sulphonates.

26. Transparent composition for the cosmetic treatment of keratin materials according to any one of the preceding claims, **characterized in that** it comprises the cationic surfactants in an amount of between 0.5 and 10% by weight relative to the total weight of the composition.

27. Transparent composition for the cosmetic treatment of keratin materials according to Claim 26, **characterized in that** it comprises the cationic surfactants in an amount of between 0.8 and 8% by weight relative to the total weight of the composition.

28. Transparent composition for the cosmetic treatment of keratin materials according to Claim 27, **characterized in that** it comprises the cationic surfactants in an amount of between 1 and 5% by weight relative to the total weight of the composition.

29. Transparent composition for the cosmetic treatment of keratin materials according to any one of the preceding claims, **characterized in that** the cosmetically acceptable medium comprises water or a mixture of water and of a cosmetically acceptable solvent.

30. Transparent composition for the cosmetic treatment of keratin materials according to Claim 29, **characterized in that** the cosmetically acceptable solvent is chosen from C₁-C₄ lower alcohols, C₅-C₁₀ alkanes, C₃₋₄ ketones, C₁-C₄ alkyl acetates, dimethoxyethane and diethoxyethane, and mixtures thereof.

31. Transparent composition for the cosmetic treatment of keratin materials according to any one of the preceding claims, **characterized in that** it comprises additives such as cationic, anionic, nonionic or amphoteric polymers, modified or unmodified nonvolatile silicones, natural or synthetic, anionic, amphoteric, zwitterionic, nonionic or cationic associative or non-associative polymeric thickeners, sunscreens, fragrances, dyes, organic or mineral particles, preservatives and pH stabilizers.

32. Process for the cosmetic treatment of keratin materials, **characterized in that** a transparent cosmetic treatment composition according to any one of the preceding claims is applied to the keratin materials.

33. Use of a transparent cosmetic treatment composition according to any one of Claims 1 to 31, as a hair conditioner.

## Patentansprüche

1. Transparente Zusammensetzung für die kosmetische Behandlung von Keratinsubstanzen, **dadurch gekennzeichnet, dass** sie in der Form einer Wasser-in-Öl-Emulsion vorliegt und **dadurch**, dass sie in einem kosmetisch akzeptabeln Medium mindestens eine Verbindung, die unter den Polyolen und anorganischen Elektrolyten ausgewählt ist, mindestens ein flüchtiges Silicon in einem Mengenanteil unter 15 Gew.-%, bezogen auf das Gesamtgewicht der Zusammensetzung, mindestens einen siliconierten grenzflächenaktiven Stoff und mindestens einen kationischen grenzflächenaktiven Stoff in einer Konzentration über 0,5 Gew.-%, bezogen auf das Gesamtgewicht der Zusammensetzung, enthält und **dadurch**, dass das Verhältnis (Polyol(e) und/oder Elektrolyt(e))/Öle größer oder gleich 2 ist, wobei die Öle das zumindest eine flüchtige Silicon umfassen.

2. Transparente Zusammensetzung für die kosmetische Behandlung von Keratinsubstanzen nach Anspruch 1, **dadurch gekennzeichnet, dass** das Verhältnis (Polyol(e) und/oder Elektrolyt(e))/Öle im Bereich von 2 bis 10 liegt.

3. Transparente Zusammensetzung für die kosmetische Behandlung von Keratinsubstanzen nach Anspruch 2, **dadurch gekennzeichnet, dass** das Verhältnis (Polyol(e) und/oder Elektrolyt(e))/Öle im Bereich von 2 bis 5 liegt.

4. Transparente Zusammensetzung für die kosmetische Behandlung von Keratinsubstanzen nach einem der vorhergehenden Ansprüche, **dadurch gekennzeichnet, dass** die Öle ferner mindestens eine Verbindung umfassen, die unter den pflanzlichen Ölen, tierischen Ölen, Mineralölen, synthetischen Ölen, Fettsäureestern und deren Gemischen ausgewählt ist.

5. Transparente Zusammensetzung für die kosmetische Behandlung von Keratinsubstanzen nach einem der vorhergehenden Ansprüche, **dadurch gekennzeichnet, dass** die Öle in einem Mengenanteil von kleiner oder gleich 20 Gew.-% enthalten sind, bezogen auf das Gesamtgewicht der Zusammensetzung.

6. Transparente Zusammensetzung für die kosmetische Behandlung von Keratinsubstanzen nach Anspruch 5, **dadurch gekennzeichnet, dass** die Öle in einem Mengenanteil im Bereich von 5 bis 20 Gew.-%, bezogen auf das Gesamtgewicht der Zusammensetzung, enthalten sind.

7. Transparente Zusammensetzung für die kosmetische Behandlung von Keratinsubstanzen nach einem der vorhergehenden Ansprüche, **dadurch gekennzeichnet, dass** die Polyole unter den Zuckern und C₃₋₂₀-Alkylenpolyolen ausgewählt sind.

8. Transparente Zusammensetzung für die kosmetische Behandlung von Keratinsubstanzen nach Anspruch 7, **dadurch gekennzeichnet**, das die Polyole unter Glycerin, Sorbit, Propylenglycol und C₈₋₂₀-Polyethylenglycolen ausgewählt sind.

9. Transparente Zusammensetzung für die kosmetische Behandlung von Keratinsubstanzen nach einem der vorhergehenden Ansprüche, **dadurch gekennzeichnet, dass** die Polyole in einem Mengenanteil von 15 bis 55 Gew.-%, bezogen auf das Gesamtgewicht der Zusammensetzung, enthalten sind.

10. Transparente Zusammensetzung für die kosmetische Behandlung von Keratinsubstanzen nach Anspruch 9, **dadurch gekennzeichnet, dass** die Polyole in einem Mengenanteil von 20 bis 50 Gew.-%, bezogen auf das Gesamtgewicht der Zusammensetzung, enthalten sind.

11. Transparente Zusammensetzung für die kosmetische Behandlung von Keratinsubstanzen nach einem der vorhergehenden Ansprüche, **dadurch gekennzeichnet, dass** die anorganischen Elektrolyte unter Natriumchlorid, Kaliumchlorid, Calciumchlorid, Magnesiumchlorid, Magnesiumsulfat und Natriumsalzen von Phosphorsäure ausgewählt sind.

12. Transparente Zusammensetzung für die kosmetische Behandlung von Keratinsubstanzen nach einem der vorhergehenden Ansprüche, **dadurch gekennzeichnet, dass** die anorganischen Elektrolyte in einem Mengenanteil von 0,1 bis 20 Gew.- %, bezogen auf das Gesamtgewicht der Zusammensetzung, enthalten sind.

13. Transparente Zusammensetzung für die kosmetische Behandlung von Keratinsubstanzen nach Anspruch 12, **dadurch gekennzeichnet, dass** die anorganischen Elektrolyte in einem Mengenanteil von 0,5 bis 15 Gew.- %, bezogen auf das Gesamtgewicht der Zusammensetzung, enthalten sind.

14. Transparente Zusammensetzung für die kosmetische Behandlung von Keratinsubstanzen nach einem der vorhergehenden Ansprüche, **dadurch gekennzeichnet, dass** die flüchtigen Silicone lineare oder cyclische Silicone sind, die bei Raumtemperatur und Atmosphärendruck eine Viskosität unter 8 mm²/s aufweisen.

15. Transparente Zusammensetzung für die kosmetische Behandlung von Keratinsubstanzen nach Anspruch 14, **dadurch gekennzeichnet, dass** die flüchtigen Silicone ausgewählt sind unter:
- cyclischen flüchtigen Siliconen mit 3 bis 7 Siliciumatomen, und
- linearen flüchtigen Siliconen mit 2 bis 9 Siliciumatomen, die bei 25 °C eine Viskosität von kleiner oder gleich 5 mm²/s aufweisen.

16. Transparente Zusammensetzung für die kosmetische Behandlung von Keratinsubstanzen nach einem der vorhergehenden Ansprüche, **dadurch gekennzeichnet, dass** sie die flüchtigen Silicone in einem Mengenanteil von 5 bis 15 Gew.-%, bezogen auf das Gesamtgewicht der Zusammensetzung, enthält.

17. Transparente Zusammensetzung für die kosmetische Behandlung von Keratinsubstanzen nach Anspruch 16, **dadurch gekennzeichnet, dass** sie die flüchtigen Silicone in einem Mengenanteil von 8 bis 15 Gew.-%, bezogen auf das Gesamtgewicht der Zusammensetzung, enthält.

18. Transparente Zusammensetzung für die kosmetische Behandlung von Keratinsubstanzen nach einem der vorhergehenden Ansprüche, **dadurch gekennzeichnet, dass** die siliconierten grenzflächenaktiven Stoffe unter den Verbindungen der allgemeinen Formeln (I), (II), (III), (IV) und (V) ausgewählt sind: 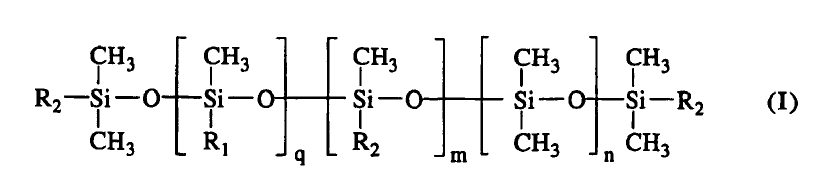 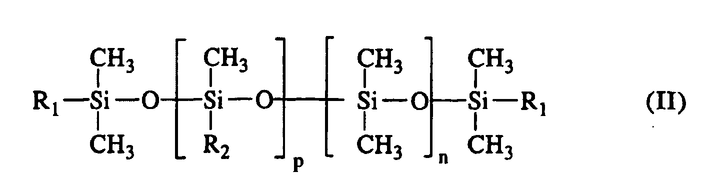 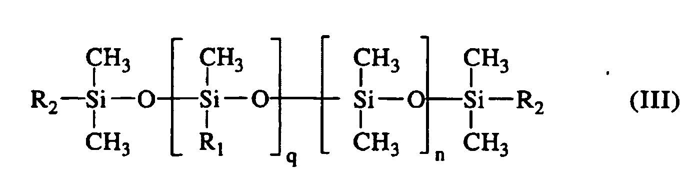 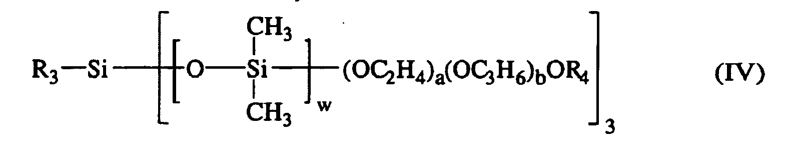 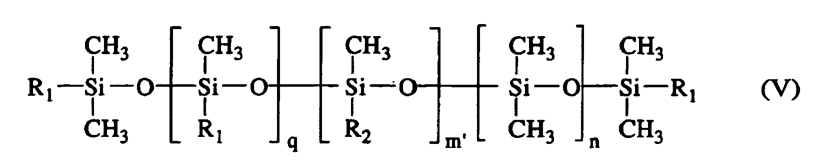 in den Formeln:
- die Gruppen R₁, die gleich oder verschieden sind, bedeuten eine geradkettige oder verzweigte Alkyl(C₁₋₃₀)gruppe oder Phenyl;
- die Gruppen R₂, die gleich oder verschieden sind, bedeuten -C_{c}H_{2c}-O-(C₂H₄O)ₐ-(C₃H₆O)_{b}-R₅ oder -C_{c}H_{2c}-O-(C₄H₈O)ₐ-R₅;
- die Gruppen R₃ und R₄, die gleich oder verschieden sind, bedeuten eine geradkettige oder verzweigte Alkyl(C₁₋₁₂)gruppe und vorzugsweise die Methylgruppe;
- die Gruppen R₅, die gleich oder verschieden sind, sind unter einem Wasserstoffatom, einer geradkettigen oder verzweigten Alkylgruppe mit 1 bis 12 Kohlenstoffatomen, einer geradkettigen oder verzweigten Alkoxygruppe mit 1 bis 6 Kohlenstoffatomen, einer geradkettigen oder verzweigten Acylgruppe mit 2 bis 12 Kohlenstoffatomen, einer Hydroxygruppe, -SO₃M, -OCOR₆, einer gegebenenfalls am Amin substituierten Aminoalkoxy(C₁₋₆)gruppe, einer gegebenenfalls am Amin substituierten Aminoacyl(C₂₋₆)gruppe, -NHCH₂CH₂COOM, -N(CH₂CH₂COOM)₂, einer gegebenenfalls am Amin und gegebenenfalls an der Alkylkette substituierten Aminoalkyl(C₁₋₁₂)-gruppe, einer Carboxyacyl(C₁₋₃₀)gruppe, einer Phosphonogruppe, die gegebenenfalls mit einer oder zwei substituierten Aminoalkyl(C₁₋₁₂)gruppen substituiert ist, -CO(CH₂)_{d}COOM, -OCOCHR₇(CH₂)_{d}COOM, -NHCO(CH₂)_{d}OH, -NH₃Y;
- die Gruppen M, die gleich oder verschieden sind, bedeuten ein Wasserstoffatom, Na, K, Li, NH₄ oder ein organisches Amin;
- R₆ ist eine geradkettige oder verzweigte Alkyl(C₁₋₃₀)gruppe;
- R₇ bedeutet ein Wasserstoffatom oder eine Gruppe SO₃M;
- d liegt im Bereich von 1 bis 10;
- m liegt im Bereich von 0 bis 20;
- m' liegt im Bereich von 1 bis 20;
- n liegt im Bereich von 0 bis 500;
- p liegt im Bereich von 1 bis 50;
- q liegt im Bereich von 0 bis 20;
- a liegt im Bereich von 0 bis 50;
- b liegt im Bereich von 0 bis 50;
- a + b ist größer oder gleich 1;
- c liegt im Bereich von 0 bis 4;
- w liegt im Bereich von 1 bis 100;
- Y bedeutet ein anorganisches oder organisches, einwertiges Anion, wie ein Halogenid, ein Sulfat, ein Carboxylat.

19. Transparente Zusammensetzung für die kosmetische Behandlung von Keratinsubstanzen nach Anspruch 18, **dadurch gekennzeichnet, dass** die siliconierten grenzflächenaktiven Stoffe unter den Verbindungen der allgemeinen Formeln (I) oder (II) ausgewählt sind, bei denen mindestens eine und vorzugsweise alle folgenden Bedingungen erfüllt sind:
- c bedeutet 2 oder 3,
- R₁ bedeutet die Methylgruppe,
- R₅ bedeutet ein Wasserstoffatom, eine Methylgruppe oder eine Acetylgruppe und vorzugsweise ein Wasserstoffatom,
- a liegt im Bereich von 1 bis 25 und insbesondere 2 bis 25,
- b liegt im Bereich von 0 bis 25,
- n liegt im Bereich von 0 bis 100,
- p liegt im Bereich von 1 bis 20.

20. Transparente Zusammensetzung für die kosmetische Behandlung von Keratinsubstanzen nach einem der vorhergehenden Ansprüche, **dadurch gekennzeichnet, dass** sie die siliconierten grenzflächenaktiven Stoffe in einem Mengenanteil von 0,01 bis 10 Gew.-%, bezogen auf das Gesamtgewicht der Zusammensetzung, enthält.

21. Transparente Zusammensetzung für die kosmetische Behandlung von Keratinsubstanzen nach Anspruch 20, **dadurch gekennzeichnet, dass** sie die siliconierten grenzflächenaktiven Stoffe in einem Mengenanteil von 0,1 bis 5 Gew.-%, bezogen auf das Gesamtgewicht der Zusammensetzung, enthält.

22. Transparente Zusammensetzung für die kosmetische Behandlung von Keratinsubstanzen nach Anspruch 21, **dadurch gekennzeichnet, dass** sie die siliconierten grenzflächenaktiven Stoffe in einem Mengenanteil von 0,2 bis 3 Gew.-%, bezogen auf das Gesamtgewicht der Zusammensetzung, enthält.

23. Transparente Zusammensetzung für die kosmetische Behandlung von Keratinsubstanzen nach einem der vorhergehenden Ansprüche, **dadurch gekennzeichnet, dass** die kationischen grenzflächenaktiven Stoffe unter den Salzen von primären, sekundären oder tertiären Aminen, die gegebenenfalls polyalkoxyliert sind, quartären Ammoniumsalzen und deren Gemischen ausgewählt sind.

24. Transparente Zusammensetzung für die kosmetische Behandlung von Keratinsubstanzen nach Anspruch 23, **dadurch gekennzeichnet, dass** die quartären Ammoniumsalze ausgewählt sind unter:
- quartären Ammoniumsalzen der folgenden allgemeinen Formel (VI): 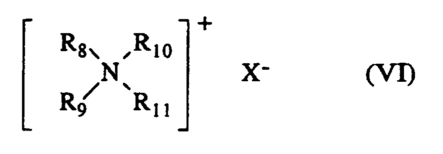 worin die Gruppen R₈ bis R₁₁, die gleich oder verschieden sein können, eine geradkettige oder verzweigte, aliphatische Gruppe mit 1 bis 30 Kohlenstoffatomen oder eine aromatische Gruppe, wie Aryl oder Alkylaryl, bedeuten; und
X ein Anion ist, das unter den Halogeniden, Phosphaten, Acetaten, Lactaten, Alkyl(C₂₋₆)sulfaten, Alkyl- oder Alkylarylsulfonaten ausgewählt ist;
- quartären Ammoniumsalzen von Imidazolin;
- quartären Diammoniumsalzen der Formel (VIII): 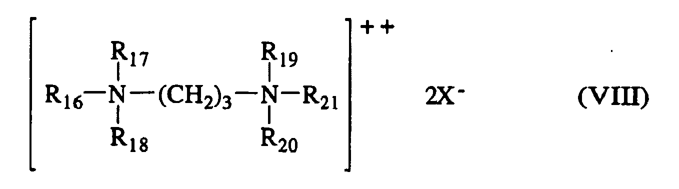 worin die Gruppe R₁₆ eine aliphatische Gruppe mit etwa 16 bis 30 Kohlenstoffatomen bedeutet, die Gruppen R₁₇, R₁₈, R₁₉, R₂₀ und R₂₁, die gleich oder verschieden sind, unter Wasserstoff oder einer Alkylgruppe mit 1 bis 4 Kohlenstoffatomen ausgewählt sind, und X ein Anion ist, das unter den Halogeniden, Acetaten, Phosphaten, Nitraten und Methylsulfaten ausgewählt ist;
- quartären Ammoniumsalzen, die mindestens eine Esterfunktion enthalten.

25. Transparente Zusammensetzung für die kosmetische Behandlung von Keratinsubstanzen nach Anspruch 23 oder 24, **dadurch gekennzeichnet, dass** die quartären Ammoniumsalze ausgewählt sind unter:
- quartären Ammoniumsalzen der folgenden allgemeinen Formel (VI): 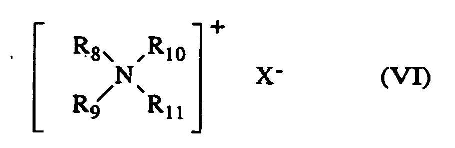 in der Formel:
R₈ bedeutet eine Alkyl(C₁₂₋₃₀)gruppe und vorzugsweise eine Alkyl(C₁₄₋₂₂)gruppe, eine Alkenyl(C₁₂₋₃₀)gruppe, eine Alkyl(C₁₂₋₂₂)-amidoalkyl(C₂₋₆)gruppe, eine Alkyl(C₁₂₋₂₂)acetatgruppe oder eine aromatische Gruppe, wie Aryl oder Alkylaryl mit 6-12C;
die Gruppen R₉ bis R₁₁, die gleich oder verschieden sein können, bedeuten eine Alkyl(C₁₋₈)gruppe, eine Alkenyl(C₁₋₈)gruppe, eine Alkoxy(C₁₋₈)gruppe, eine Hydroxyalkyl(C₁₋₈)gruppe, eine Polyoxyalkylen(C₂₋₆)gruppe oder eine Alkylamid(C₁₋₈)gruppe;
X ist ein Anion, das unter den Halogeniden, Phosphaten, Acetaten, Lactaten, Alkyl(C₂₋₆)sulfaten, Alkyl- oder Alkylarylsulfonaten ausgewählt ist.

26. Transparente Zusammensetzung für die kosmetische Behandlung von Keratinsubstanzen nach einem der vorhergehenden Ansprüche, **dadurch gekennzeichnet, dass** sie die kationischen grenzflächenaktiven Stoffe in einem Mengenanteil von 0,5 bis 10 Gew.-%, bezogen auf das Gesamtgewicht der Zusammensetzung, enthält.

27. Transparente Zusammensetzung für die kosmetische Behandlung von Keratinsubstanzen nach Anspruch 26, **dadurch gekennzeichnet, dass** sie die kationischen grenzflächenaktiven Stoffe in einem Mengenanteil von 0,8 bis 8 Gew.-%, bezogen auf das Gesamtgewicht der Zusammensetzung, enthält.

28. Transparente Zusammensetzung für die kosmetische Behandlung von Keratinsubstanzen nach Anspruch 27, **dadurch gekennzeichnet, dass** sie die kationischen grenzflächenaktiven Stoffe in einem Mengenanteil von 1 bis 5 Gew.-%, bezogen auf das Gesamtgewicht der Zusammensetzung, enthält.

29. Transparente Zusammensetzung für die kosmetische Behandlung von Keratinsubstanzen nach einem der vorhergehenden Ansprüche, **dadurch gekennzeichnet, dass** das kosmetisch akzeptable Medium Wasser oder ein Gemisch von Wasser und einem kosmetisch akzeptablen Lösemittel enthält.

30. Transparente Zusammensetzung für die kosmetische Behandlung von Keratinsubstanzen nach Anspruch 29, **dadurch gekennzeichnet, dass** das kosmetisch akzeptable Lösemittel unter den niederen C₁₋₄-Alkoholen, C₅₋₁₀-Alkanen, C₃₋₄-Ketonen, Alkyl(C₁₋₄)acetaten, Dimethoxyethan, Diethoxyethan und deren Gemischen ausgewählt ist.

31. Transparente Zusammensetzung für die kosmetische Behandlung von Keratinsubstanzen nach einem der vorhergehenden Ansprüche, **dadurch gekennzeichnet, dass** sie Additive enthält, wie kationische, anionische, nichtionische oder amphotere Polymere, nicht flüchtige, gegebenenfalls modifizierte Silicone, natürliche oder synthetische, anionische, amphotere, zitterionische, nichtionische oder kationische, assoziative oder nicht assoziative, polymere Verdickungsmittel, Sonnenschutzfilter, Parfums, Farbmittel, organische oder anorganische Partikel, Konservierungsmittel, pH-Stabilisatoren.

32. Verfahren zur kosmetischen Behandlung von Keratinsubstanzen, **dadurch gekennzeichnet, dass** auf die Keratinsubstanzen eine transparente Zusammensetzung für die kosmetische Behandlung nach einem der vorhergehenden Ansprüche aufgetragen wird.

33. Verwendung einer transparenten Zusammensetzung für die kosmetische Behandlung nach einem der Ansprüche 1 bis 31 als Produkt, das nach der Haarwäsche angewandt wird.
